# EUROPEAN PATENT SPECIFICATION

(11) **EP 0 824 026 B1**
(45) Date of publication and mention of the grant of the patent: **30.11.2005**
(21) Application number: 97305999.1
(22) Date of filing: 07.08.1997
(51) Int. Cl.: A61N 5/10, A61B 6/08

(54) **Radiation treatment apparatus and method**
Bestrahlungsbehandlungsvorrichtung und Verfahren
Dispositif de radiothérapie et procédé

(30) Priority: 12.08.1996 US 695457
(43) Date of publication of application: 18.02.1998
(73) Proprietor: Siemens Medical Solutions USA, Inc., Iselin, NJ 08830 (US)
(72) Inventor: Hughes, John, Martinez, CA 94553 (US)
(74) Representative: Fenlon, Christine Lesley

(56) References cited:
- EP-A- 0 314 231
- US-A- 5 142 559

## Description

The invention relates to an apparatus for radiation treatment and a method of operating the radiation treatment apparatus and, more especially, to the definition of the radiation field in the treatment zone.

A radiation treatment apparatus is known which comprises a gantry which can be swivelled around a horizontal axis of rotation in the course of a therapeutic treatment and a linear accelerator located in the gantry for generating a high-energy radiation beam for therapy. The high energy radiation beam can be an electron beam or an electromagnetic beam, such as an X-ray beam. During treatment, the radiation beam is directed onto a part of a patient lying in the isocentre of the gantry rotation.

To control the radiation associated with the beam, it is known to provide a beam-shielding device, such as a plate arrangement and/or collimator, in the trajectory of the radiation beam between the radiation source and the region in which an object is placed for treatment. The beam-shielding device defines a field on the object to be irradiated with a prescribed amount of radiation. The size of the field is significant in that it defines the region of a patient that will be irradiated.

The radiation delivered to an object may be analyzed into primary and secondary components. The primary radiation is made up of the initial or original electromagnetic radiation emitted from the radiation source. The secondary radiation is the result of the photons scattered by the plate arrangement. The beam's radiation output in free space increases because of the increased plate-collimator scatter, which is added to the primary beam. In other words, a point in the field is subject not only to the primary, direct radiation but also the secondary radiation scattered from the plate arrangement. The ratio of the radiation output in air with the scatterer to the radiation output without the scatterer for a reference field (e.g. of size 10 x 10 cm) is commonly called the "output factor" or the "collimator scatter factor." The concept and definition of the output factor are well understood in the art.

Owing to the secondary radiation from the scattered photons, the dose rate applied to the surface of an object is dependent on the size of the opening in the plate arrangement, i.e. on the field size. This means that the irradiation level at a specific spot on an object, for instance a spot in the centre of the radiation beam, changes according to the size of the opening in the plate arrangement. In general, when the plate arrangement has a relatively small opening, the accumulated dose at a specific spot will be less than when the opening is relatively large.

To set the field size, a radiation treatment device has a set-up mode in which light visible to the operator is projected from the treatment head to form a visible light field on the patient. The visible light field facilitates the adjustment of beam parameters and the positioning of the patient relative to the treatment head. For the adjustment, the field size of the visible light field is taken to be indicative of the field size of the radiation beam that will pertain during subsequent treatment.

The field size is adjusted by varying an aperture through a collimator in the treatment head. The aperture is defined by settings of X-axis collimator jaws and Y-axis collimator jaws, the jaws being blocks of radiation-attenuating material that determine the field size by limiting the angular spread of the beam. By convention, the X-axis jaws are located below the Y-axis jaws.

The set-up procedure is thus based on the presumption that the field size and location of the radiation beam will coincide with those of the visible light. However, there is not in fact an exact coincidence between the respective fields, since the propagation and other characteristics of the treatment radiation and visible light, such as intensity, spot size and position, are significantly different. Moreover, in the case of X-rays, at different X-ray energies the treatment radiation beam will have different scattering components. The penumbra of the two field edges will be dissimilar. As a consequence, if the collimator jaws are adjusted during a set-up procedure using visible light, the field size for the X-ray beam used for treatment may be significantly different.

In order to decrease the difference between the sizes of the generally larger light field and the radiation field, so-called trimmers may be attached to the edges of the jaws. Trimmers are formed of a material, such as aluminium, that is transparent to X-ray radiation but opaque to visible light. Trimmers thus serve at least to decrease the size difference between the visible light field and the X-ray radiation field. The trimmers may be X-ray transparent blades that project slightly (e.g. 4.3 mm) beyond the faces of collimator jaws to trim the light field. However, the phenomena that create the non-coincidence are partially dependent upon the energy of the radiation.
Consequently, trimmers of dimensions suitable for providing coincidence at a relatively low energy level (e.g. 6 MV) will be less effective in establishing coincidence if the apparatus is reset to operate at a higher energy level (e.g. 23 MV). A compromise may be selected by using trimmers having a width suitable for providing coincidence at the middle of the available range of energies. Field size coincidence will however worsen towards the high and low ends of the energy capabilities of the apparatus. Thus, unless trimmers are changed with almost each change in the desired treatment beam parameters, a high level of field size coincidence cannot be achieved.

Patent document EP 0314231 (Philips Electronic et al.) discloses a collimating arrangement for use in radiotherapy apparatus which has a set of radiation attenuating members. Each member is provided with a retroreflector for reflecting illumination from a lamp into a camera. Thus the camera detects the positions of each member.

According to an aspect of the invention, there is provided a method of operating a radiation treatment apparatus having a field-defining structure, wherein a setting of the field-defining structure defines a dimension of a light field formed in a set-up mode of the system and a corresponding dimension of a radiation field formed in an operational mode of the apparatus, the method comprising automatically adjusting the setting of the field-defining structure when switching between the setup mode and the operational mode, the adjusting configured to compensate for the difference in dimension between the light field and the radiation field which would otherwise occur on mode switching if the setting of the field-defining structure remained unchanged.

According to another aspect of the invention, there is provided a method of determining dimensional data for a radiation treatment apparatus having a field-defining structure, wherein a setting of the field-defining structure defines a dimension of a light field formed in a set-up mode of the apparatus and a corresponding dimension of a radiation field formed in an operational mode of the apparatus, the method comprising the steps of determining, for a plurality of settings of the field-defining structure, data indicative of the difference between the respective dimensions of the light field and the radiation field formed at that setting; and storing said data as said dimensional data.

According to a further aspect of the invention, there is provided a radiation treatment apparatus comprising: a radiation source (26) for generating a radiation beam; means (30, 34) for manipulating the radiation beam to define a radiation field in a treatment zone (22); a light source (52) for generating a light beam; means for manipulating the light beam to define a light field in the treatment zone (22); a field-defining structure (36) common to the radiation and light beam manipulating means and arranged in the path of the light beam and the radiation beam, a setting of the field-defining structure (36) defining a dimension of the light field and a corresponding dimension of the radiation field and means for switching the radiation apparatus between a set-up mode in which a light field is formed in the treatment zone and an operational mode in which a radiation field is formed in the treatment zone; characterized by means (28, 60; 92) for automatically adjusting the setting of the field-defining structure (36) when switching between the set-up mode and the operational mode, the adjusting configured to compensate for the difference in the dimension between the light field and the radiation field which would otherwise occur in mode switching if the setting of the field-defining structure remained unchanged.

Further aspects of the invention are exemplified by the attached claims.

For a better understanding of the invention, and to show how the same may be carried into effect, reference will now be made, by way of example, to the accompanying drawings, in which:-
Fig. 1 is a perspective view of a radiation apparatus for providing automated field size coincidence;
Fig. 2 is a block diagram of the radiation apparatus of Fig. 1;
Fig. 3 is a schematic representation of field-defining structure for the collimator of Fig. 2;
Fig. 4 illustrates one embodiment of a process flow for implementing automated field size coincidence;
Fig. 5 is a table of field sizes that are calculated and stored during implementation of the process of Fig. 4; and
Fig. 6 shows a parabolic mirror of an alternative embodiment.

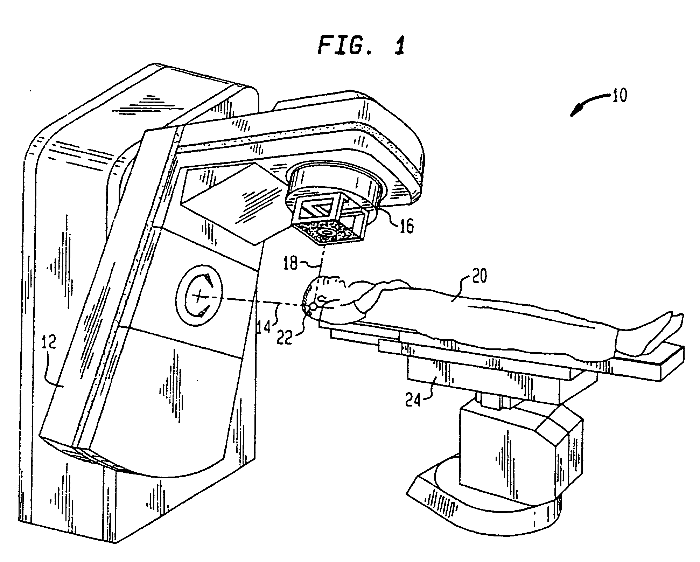

Fig. 1 is a perspective view of a radiation apparatus 10 for medical applications and includes a gantry 12 which can be swivelled around a horizontal axis of rotation 14 in the course of a therapeutic treatment. A treatment head 16 of the gantry 12 directs a radiation beam along an axis 18 toward a patient 20. The radiation beam is generated by a linear accelerator arranged within the gantry. The radiation beam may be electron radiation or electromagnetic radiation such as X-ray radiation. The radiation beam is trained on a treatment zone 22 of the patient.

The treatment parameters of a particular therapeutic session are defined when the radiation apparatus 10 is in a set-up mode. The treatment zone 22 is positioned relative to the gantry 12 by rotating the gantry about the horizontal axis 14 and by moving a treatment table 24 on which the patient 20 rests. After the treatment zone has been positioned, beam parameters are set. Preferably, the radiation apparatus 10 allows one of a number of energy levels to be selected, such as X-ray energy levels of 6 MV, 15 MV and 23 MV. The dimensions of the radiation field should match the dimensions of the treatment zone 22, so that only that region of the patient that is to be treated will be exposed to radiation. As will be explained more fully below, the dimensions of the radiation field are determined by field-defining structure within the treatment head 16. During the set-up stage, the beam that is projected along axis 18 is a beam of visible light that allows a user to non-intrusively adjust the aim and the dimensions of the beam that is projected along the axis 18. A projection lamp may be activated during the set-up mode of the apparatus to provide the desired light field at the treatment zone 22. When the apparatus is switched to the operational mode, the visible light is replaced with the radiation beam for treatment.

A conventional linear accelerator ("linac") 26 may be used to generate an electron beam, as the radiation beam, emitted from the radiation apparatus 10 of Fig.1. The energy level of the electron beam is determined by a controller 28 that activates an electron gun of the linac 26. The electrons from the electron gun are accelerated along a waveguide using known energy-transfer techniques. The controller 28 is also arranged in communication with a data store 60, the role of which is described further below.

The electron beam from the waveguide of the linac 26 enters a conventional guide magnet 30, which bends the electron beam by approximately 270°. The electron beam then exits through a window that is transparent to the beam, but allows a vacuum to be maintained within the linac 26.

Along an axis 32 of the electron beam emitted from the guide magnet 30 there is positioned a scattering foil or a target 34. If the element 34 is a scattering foil, the electrons are spread to form a conical electron beam. If the element 34 is a target, the radiation beam is transformed into X-rays.

A collimator 36 is positioned along the radiation beam path and limits the angular spread of the radiation beam. For example, blocks of radiation-attenuating material may be used to define a radiation field that passes through the collimator to a receptor 38. The receptor may be the patient or a structure used to calibrate the radiation apparatus during a calibration process. A monitor or display 66 is arranged in communication with the linac controller 28 and the receptor 38. The use of the monitor 66 is described further below.

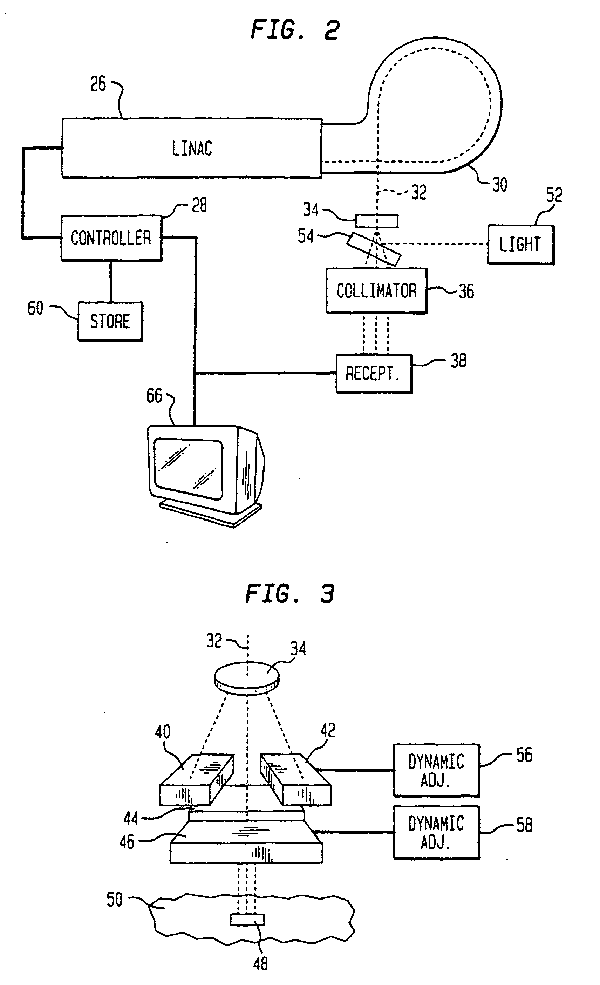

Figure 3 shows in more detail the target 34 and collimator 36.

The collimator 36 includes Y-axis jaws and X-axis jaws. In Fig. 3, the Y-axis jaws are represented by first and second blocks 40 and 42 of radiation-attenuating material. Below the Y-axis jaws are arranged third and fourth blocks 44 and 46 that form the X-axis jaws. The spacing between the first and second blocks defines one dimension of the target zone 48 on a body 50, while the spacing between the third and fourth blocks defines a further dimension generally at right-angles to the first-mentioned dimension. The blocks 40, 42, 44 and 46 thus define the field and the dimensions of the target zone 48, and are referred to as "field-defining structure".

In the set-up mode of the radiation apparatus, the linac 26 is deactivated and the light source 52 is energized. The light source directs visible light to an optical element 54, such as a beam splitter, for redirecting the light into the collimator 36. The light source may for example be a 150 W quartz halogen lamp. The optical element 54 is transparent to radiation from the linac 26. When the light has passed through the collimator, the beam can be used to position the jaws of the collimator. For example, if the target zone 48 on the body 50 of Fig. 3 is a tattooed area on a patient, the blocks 40-46 may be adjusted until the light field that is emitted from the collimator coincides with the area of the target zone. The radiation apparatus can then be switched to an operational mode in which the radiation beam takes the place of the light beam.

A pair of dynamic adjusters 56 and 58 are provided for controlling the X-axis and Y-axis jaws respectively and serve to allow automatic adjustment of the settings of the respective jaws to compensate for inherent dissimilarities between field sizes of the visible light beam and the treatment radiation beam. The apparatus may allow for adjustment of the X-axis and Y-axis jaws when the light is turned on.

The dynamic adjusters 56 and 58 are arranged to receive control signals from the controller 28. In a preferred example, the increments of jaw adjustment are defined according to the content of one or more look-up tables stored at the store 60 and accessed by control software for setting the "trimming" of the light field. For a given energy level of X-ray radiation, say 15 MV, and for a desired field dimension, say 10 cm, data stored in the look-up table will indicate that to obtain an X-ray field dimension of 10 cm the appropriate jaw dimension should be set to 10.2 cm during the set-up mode using a visible optical beam. The dynamic adjusters 56 and 58 can then be activated by the controller 28 to automatically vary the positioning of the blocks 40-46 to compensate for the difference of 0.2 cm between inherent field sizes of the optical and X-ray radiation.

The dynamic adjusters 56 and 58 may be constituted by any electronically controllable device capable of manipulating the blocks 40-46.

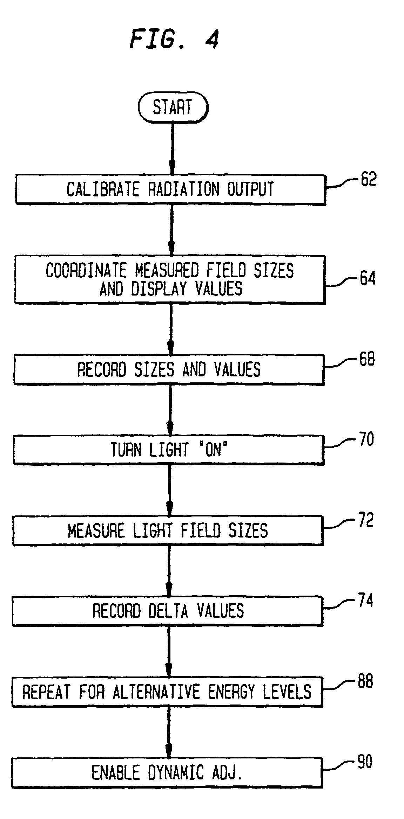

Fig. 4 illustrates one embodiment of a process for establishing coincidence between field sizes of a light field and a radiation field for the apparatus 10 of Fig. 1. In step 62, the collimator field size is calibrated for the radiation output. Calibration of a radiation apparatus for X-ray output is well known in the art, and any of the known techniques may be utilized in executing step 62. For example, a tank of water may be used to simulate a human body or other object and a probe may be used to measure radiation through the water. With the water surface at a 900 mm target-surface distance (TSD) and the probe at the isocentre, the field size may be measured for a particular setting of the jaws. Conventionally, the field size is measured with regard to the fifty percent maximum dose value of the radiation. This measurement is stored at element 60 of Fig. 2. The procedure is repeated for a number of settings of the jaws, and each measurement is recorded. Preferably, the energy level of the radiation and the TSD are constant throughout the calibration, since these two factors affect scattering and diffraction of the beam. However, as will be explained more fully below, the process steps preferably are carried out with regard to more than one energy level and/or with regard to more than one TSD.

In step 64, the measurements of field sizes acquired during step 62 are coordinated with values on the display 66 (see Fig. 2) that is employed by a user of the radiation apparatus. The display will include designations of dimensions. In the exemplary embodiment of Fig. 4, the indications are manipulable, so that the display values can be coordinated with the measured field sizes at each of the various settings of the jaws. In step 68, the data from steps 62 and 64 are recorded.

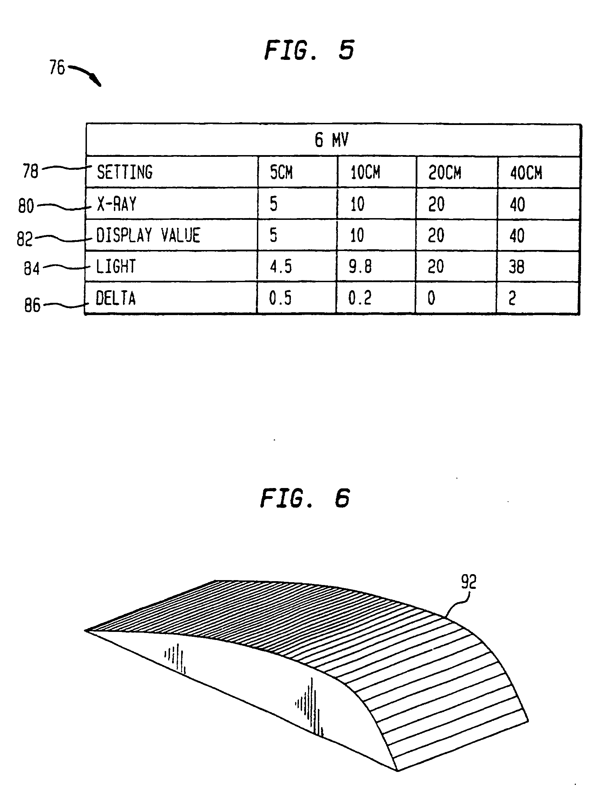

The light 52 of Fig. 2 is then turned "on," as shown in step 70 of Fig. 4. For the same jaw settings that were used in the calibration of the radiation output at step 62, the light field sizes are measured in step 72. This may be done using conventional techniques. For example, the tank of water used in step 62 may be replaced with graph paper or with a film pack. The portion of the graph paper or film that is illuminated by the light beam is measured for each setting. Next, the dimensional difference between the light field and the radiation field is determined for each jaw setting. The delta values are recorded at step 74. In one embodiment, the delta value is merely a difference between the measured light field size from step 72 and the display monitor value for the particular setting of the jaws. Because the phenomena of scattering and diffraction will have different effects upon the X-ray beam and light beam, the delta values will at least partially be indications of the different effects. Following step 74, a table may be formed from the data stored at component 60 of Fig. 2. Such a table 76 is shown in Fig. 5.

Within step 70, the first row indicates the various settings of the jaws that affect the field size of concern. That is, the first row is an indication of the setting 78 of the field-defining structure that can be dynamically adjusted. The second row 80 shows the measured field sizes for the X-ray field at an energy level of 6 MV. The values of rows 78 and 80 are identical, since the radiation output was calibrated at step 62.

In the third row 82, the display values of the display 66 have been recorded. Since the X-ray field sizes and the display values were coordinated at step 64 of Fig. 4, the values of row 82 are identical to the values of row 80. With the X-ray output turned "off" and the light 52 turned "on", the light field sizes were measured at step 72 and recorded in the fourth row 84 of table 76. The final row 86 records the delta values that are the dimensional differences between the X-ray field size of row 80 and the light field size of row 84.

In step 88 of Fig. 4, the steps for generating the table 76 may be repeated for alternative energy levels of the radiation apparatus.

Since the radiation field sizes may vary depending upon the energy level of the radiation beam, there are potentially different delta values for the different energies. By generating separate tables that are implemented based upon the setting of energy levels, the dynamic compensation process is not a process that requires significant compromise, as would be the case if aluminium micro-trimmers were used to achieve field size coincidence.

The dynamic adjusters 56 and 58 are enabled at step 90 of the process of Fig. 4. Then, in the operation of the radiation apparatus 10 of Figs 1-3, a user selects a setting of the field-defining structure (i.e. of the blocks 40-46) in the conventional manner by forming a visible light field on a target zone on the patient 20, which may for example be delineated by a tattoo. The light field is provided by activating a light source 52 to pass light through the collimator 36. The adjustment of the field-defining structure is accomplished during the set-up mode of the radiation apparatus.

Once the blocks 40-46 have been set with respect to the dimensions of the light field, the apparatus may be switched to the operational mode. The controller 28 may provide the switching capability. As part of the mode switching, the dynamic adjusters 56 and 58 are adjusted by an increment defined by the appropriate delta value in row 86 of Fig. 5. For example, if the light field that was established during the set-up mode had a field size that spaced the first block 40 away from the second block 42 by a distance of 4.5 cm, the dynamic adjustment will be to implement an incremental increase of 0.5 cm to move the blocks apart to form a spacing of 5 cm. The incremental adjustment is executed automatically, typically in control software, so that the user is not required to provide further adjustments. Based upon the data of table 76, the controller 28 is able to interpolate and/or extrapolate the information in order to provide the appropriate compensation for a setting that is not contained within stored memory.

While the preferred embodiment of Fig. 4 uses the X-ray output to calibrate the apparatus in steps 62 and 64, there may be some applications in which the light field is used in the calibration process. Moreover, non-medical applications may also be performed.

As an alternative to the computer-based control apparatus described above, field size coincidence may be achieved using additional optical components.

One such optical embodiment has a curved mirror 92 to compensate for the dimensional error between the light field size and the radiation field size. It has been determined that an asymmetrically shaped mirror is preferred, most especially a parabolic mirror. The equation for the profile of a parabolic mirror and the positioning of the mirror relative to a target area has the form:${\text{f(x) = Ax}}^{\text{2}} \text{+ Bx + C}$ wherein the coefficient "A" describes the curvature of the mirror, "B" is a tilt term, and "C" is an offset term that by convention determines a vertical position. When the term "A" is 0, the mirror is flat, while a negative value describes a convex surface.

The mirror is preferably asymmetrical, because the intersection of the light with the mirror for a given field size is not equally spaced from the collimator axis when comparing opposite jaws. Therefore, one side of the mirror beneficially has a greater curvature. A parabolic mirror 92 is shown in Fig. 6, the curvature being exaggerated for the purpose of illustration. The actual curvature is more subtle, for example approximately 0.002 to 0.003 cm per cm.

This mirror design works well with positive field sizes, but increases the error if one of the collimator jaws defines a negative field size, i.e. if both of the blocks of the jaw are on the same side of a midline. Moreover, the mirror does not rotate with the collimator, so that this approach to correcting the light penumbra only works with the collimator at zero degrees. When the collimator is at ninety degrees, there is no correction. A three-dimensional profile could be provided to overcome this limitation.

Another possible solution is to provide a more compact light source and a second mirror, with the second mirror being placed in the head area of the radiation apparatus. By adding an elliptical reflector behind a conventional light source that produces approximately 20 lux at the isocentre, the luminance may increase to 100 lux. This increase in luminance reduces the perceived light field penumbra without an increase in the cost of the light source. However, the uniformity at the edges of the light field is obtained at the cost of creating a dark spot at the beam axis, since the lamp blocks some of the light reflected by the elliptical reflector. Frosted glass or some other diffusing means may be provided to overcome this limitation, but the diffusion will tend to reduce the efficiency.

Alternate light sources provide some benefits. For example, an arc lamp may be used in place of the conventional tungsten-filament, halogen-filled, quartz lamp. The arc lamp provides a significantly greater illuminance. When focused on a small aperture, no field size compensation was required. Because of size and also for safety reasons, the best location for an arc lamp and its power supply is in the machine stationary structure. Light guidance such as a fibre optic bundle can then be employed to bring the light to the head.

Another alternative light source is a laser. A laser has the advantage of producing a small and well-collimated beam which can be focused by a converging lens through a very small aperture to produce little or no penumbra. To achieve a 40 lux, 50 cm diameter field requires 8 lumens of luminous power. At the wavelength of a typical HeNe laser, one watt of power is approximately equal to 250 lumens so that a laser having a power of 32 mW laser would be required.

As previously noted, fibre optics may be utilized. Because there is limited space within the head area of the radiation device, the application of fibre optics is attractive. When optical fibre is used, the design aim is to keep efficiency sufficiently high to provide at least equal illumination to that of a comparable apparatus which does not use such fibre components.

Another approach is to provide a light source on the target slide of the radiation device. The advantage of this approach is that the a mirror is not required and the alignment procedure is simplified. The space that the mirror occupies in a conventional radiation device can then be used for shielding material, automated wedges, or the like. To implement this approach, the light source occupies a new position on the target slide and the target slide is movable between a set-up mode position and an operational mode position for treatment of a patient. Since the thickness of a conventional target slide may prevent placement of the light source directly on the slide, a target slide of greater structural integrity may be provided or a remote light source can be provided that is optically coupled via a fibre optic bundle.

## Claims

1. A method of operating a radiation treatment apparatus having a field-defining structure, wherein a setting of the field-defining structure defines a dimension of a light field formed in a set-up mode of the system and a corresponding dimension of a radiation field formed in an operational mode of the apparatus, the method comprising automatically adjusting the setting of the field-defining structure when switching between the setup mode and the operational mode, the adjusting configured to compensate for the difference in dimension between the light field and the radiation field which would otherwise occur on mode switching if the setting of the field-defining structure remained unchanged.

2. A method according to claim 1, wherein said automatic adjustment is made on the basis of dimensional data stored by the system, said dimensional data being indicative of said dimensions and their corresponding dimensions as a function of the setting of the field-defining structure.

3. A method according to claim 2, wherein the dimensional data comprises a plurality of difference values indicative of the respective differences between the dimensions and their corresponding dimensions.

4. A method according to claim 3, wherein the automatic adjustment is performed by adjusting the setting of the field-defining structure by an amount defined by one of said difference values.

5. A method according to claim 3, wherein the automatic adjustment is performed by adjusting the setting of the field-defining structure by an amount defined by interpolation between at least two of said difference values.

6. A method according to any one of the preceding claims, wherein the system is such that in the operational mode one of a plurality of radiation energies may be operative, the method being such that the automatic adjustment of the setting of the field-defining structure takes account of the radiation energy.

7. A method according to claim 6 when appended to claim 2, wherein the dimensional data comprises a plurality of data subsets for respective ones of the radiation energies.

8. A method according to any one of the preceding claims, wherein the automatic adjustment of the field-defining structure includes automatic adjustment of the jaws of a collimator that is connected to a linear accelerator.

9. A method of determining dimensional data for a radiation treatment apparatus having a field-defining structure, wherein a setting of the field-defining structure defines a dimension of a light field formed in a set-up mode of the apparatus and a corresponding dimension of a radiation field formed in an operational mode of the apparatus, the method comprising the steps of:
determining, for a plurality of settings of the field-defining structure, data indicative of the difference between the respective dimensions of the light field and the radiation field formed at that setting; and
storing said data as said dimensional data.

10. A method according to claim 9, wherein the determination of the dimensional data includes calibrating the radiation field at the plurality of settings of the field-defining structure and comparing the light field to results of calibrating the radiation field.

11. A method according to claim 10, wherein the calibration of the radiation field includes, at each of the settings, adjusting a displayed value of the field size of the radiation field to correspond to a measured value of the field size of the radiation field.

12. A method according to claim 9, 10 or 11, wherein said dimensional data comprises for each said setting a difference between a dimension of the light field and the corresponding dimension of the radiation field.

13. A method according to claim 10 or 11 and 12, wherein the comparison includes, at each of said settings, determining the difference between the displayed value of the field size and a measured value of the field size of the light field, thereby providing a basis for determining said difference between the light field and the corresponding radiation field at each of said settings of the field-defining structure.

14. A method according to claim 12 or 13, wherein determination of said difference includes forming and storing the dimensional data such that the settings of the field-defining structure are associated with respective difference values indicative of the difference between the measured field sizes of the corresponding light and radiation fields.

15. A method according to claim 14, wherein the dimensional data is stored in tabular form.

16. A method according to any one of claims 9 to 15, applicable to a radiation apparatus in which the operational mode is operative with one of a plurality of radiation energies so that for any dimension of the light field formed in the set-up mode there is in the operational mode a plurality of corresponding dimensions of the radiation field for the different radiation energies, wherein
the determination of the dimensional data for a plurality of settings of the field-defining structure comprises determining data indicative of the respective dimensions of the light field and a plurality of corresponding radiation fields formed at respective radiation energies.

17. A method according to claim 2 to 5, 7 and 8, and claim 6 when appended to claim 2, wherein the dimensional data is determined according to the method of any one of claims 9 to 16.

18. A method according to any one of the preceding claims, wherein said radiation field is an X-ray radiation field.

19. A radiation treatment apparatus comprising:
a radiation source (26) for generating a radiation beam;
means (30, 34) for manipulating the radiation beam to define a radiation field in a treatment zone (22);
a light source (52) for generating a light beam;
means for manipulating the light beam to define a light field in the treatment zone (22);
a field defining structure (36) common to the radiation and light beam manipulating means and arranged in the path of the light beam and the radiation beam, a setting of the field defining structure (36) defining a dimension of the light field and a corresponding dimension of the radiation field; and
means for switching the radiation apparatus between a set-up mode in which a light field is formed in the treatment zone and an operational mode in which a radiation field is formed in the treatment zone;
**characterized by**
means (28, 60; 92) for automatically adjusting the setting of the field-defining structure (36) when switching between the set-up mode and the operational mode, the adjusting configured to compensate for the difference in the dimension between the light field and the radiation field which would otherwise occur in mode switching if the setting of the field-defining structure remained unchanged.

20. An apparatus according to claim 19, wherein the automatic adjustment means (28) has storage means (60) in which are stored dimensional data indicative of said dimensions and their corresponding dimensions as a function of the setting of the field-defining structure.

21. An apparatus according to claim 20, wherein the dimensional data comprises a plurality of difference values indicative of the respective differences between the dimensions and their corresponding dimensions.

22. An apparatus according to claim 21, wherein the automatic adjustment means is operable to adjust the setting of the field-defining structure by an amount defined by one of said difference values.

23. An apparatus according to claim 21, wherein the automatic adjustment means is operable to adjust the setting of the field-defining structure by an amount defined by interpolation between at least two of said difference values.

24. An apparatus according to any one of claims 19 to 23, operable to define a radiation field having one of a plurality of radiation energies, the automatic adjustment means being operable to perform the setting of the field-defining structure taking account of the radiation energy.

25. An apparatus according to claim 24 when appended to claim 20, wherein the dimensional data comprises a plurality of data subsets for respective ones of the radiation energies.

26. An apparatus according to any one of claims 19 to 25, wherein the field defining structure comprises means (40, 42) for defining said dimension and corresponding dimension, and means (44, 46) for defining a further dimension of the light field and a corresponding further dimension of the radiation field.

27. An apparatus according to claim 26, wherein said dimension defining means comprise two pairs of jaws (40, 42 and 44, 46), one pair for each dimension defining means, the pairs of jaws being arranged to extend transverse to each other and having respective means (56, 58) for adjusting the separation between the jaws of the pair concerned.

28. An apparatus according to any one of claims 19 to 27 and comprising a display (66) for displaying an image of the radiation field and an indication of the anticipated size of the radiation field at the current setting of the field-defining structure.

29. An apparatus according to any one of claims 19 to 28, wherein the radiation beam is a beam of X-ray radiation and the radiation beam manipulating means comprises an X-ray collimator, the light and radiation beam manipulating means being arranged so that the respective paths of the light beam and the X-ray beam are aligned to direct the light beam along an axis that is generally coaxial with that of the X-ray radiation beam and through said field-defining structure (36).

30. An apparatus according to any one of claims 19 to 29, wherein the automatic adjustment means comprises a curved mirror (92).

31. An apparatus according to claim 30, wherein the curved mirror (92) has a parabolic profile.

## Patentansprüche

1. Verfahren zum Betreiben einer Strahlenbehandlungseinrichtung, die eine Feldabgrenzungsanordnung aufweist, wobei eine Einstellung der Feldabgrenzungsanordnung eine Abmessung eines Lichtfeldes festlegt, das in einem Einstellmodus des Systems ausgebildet wird, und eine entsprechende Abmessung eines Strahlungsfeldes festlegt, das in einem Betriebsmodus der Einrichtung ausgebildet wird, wobei das Verfahren umfasst, automatisch die Einstellung der Feldabgrenzungsanordnung anzupassen, wenn zwischen dem Einstellmodus und dem Betriebsmodus umgeschaltet wird, und die Anpassung so ausgebildet ist, dass die Differenz der Abmessungen zwischen dem Lichtfeld und dem Strahlungsfeld kompensiert wird, die anderenfalls bei der Modusumschaltung aufträte, wenn die Einstellung der Feldabgrenzungsanordnung unverändert bliebe.

2. Verfahren nach Anspruch 1, bei welchem die automatische Anpassung auf Grundlage von Abmessungsdaten durchgeführt wird, die von dem System gespeichert werden, und die Abmessungsdaten die Abmessungen und deren entsprechende Abmessungen als Funktion der Einstellung der Feldabgrenzungsanordnung anzeigen.

3. Verfahren nach Anspruch 2, bei welchem die Abmessungsdaten mehrere Differenzwerte umfassen, welche die jeweiligen Differenzen zwischen den Abmessungen und ihren entsprechenden Abmessungen anzeigen.

4. Verfahren nach Anspruch 3, bei welchem die automatische Anpassung **dadurch** durchgeführt wird, dass die Einstellung der Feldabgrenzungsanordnung um ein Ausmaß angepasst wird, das durch eine der Differenzwerte festgelegt wird.

5. Verfahren nach Anspruch 3, bei welchem die automatische Anpassung **dadurch** durchgeführt wird, dass die Einstellung der Feldabgrenzungsanordnung um ein Ausmaß angepasst wird, das durch Interpolation zwischen zumindest zweien der Differenzwerte festgelegt ist.

6. Verfahren nach einem der voranstehenden Ansprüche, bei welchem das System so ausgebildet ist, dass in dem Betriebsmodus eine von mehreren Strahlungsenergien betriebsfähig ist, wobei das Verfahren so ausgebildet ist, dass die automatische Anpassung der Einstellung der Feldabgrenzungsanordnung die Strahlungsenergie berücksichtigt.

7. Verfahren nach Anspruch 6 in Abhängigkeit von Anspruch 2, bei welchem die Abmessungsdaten mehrere Datenuntergruppen für die jeweilige Strahlungsenergie umfassen.

8. Verfahren nach einem der voranstehenden Ansprüche, bei welchem die automatische Anpassung der Feldabgrenzungsanordnung die automatische Anpassung eines Kollimators umfasst, der mit einem Linearbeschleuniger verbunden ist.

9. Verfahren zur Bestimmung von Abmessungsdaten für eine Strahlenbehandlungseinrichtung, die eine Feldabgrenzungsanordnung aufweist, wobei eine Einstellung der Feldabgrenzungsanordnung eine Abmessung eines Lichtfeldes festlegt, das in einem Einstellmodus der Einrichtung ausgebildet wird, und eine entsprechende Abmessung eines Strahlungsfeldes, das in einem Betriebsmodus der Einrichtung ausgebildet wird, wobei das Verfahren folgende Schritte umfasst: Bestimmung, für mehrere Einstellungen der Feldabgrenzungsanordnung, von Daten, welche die Differenz zwischen jeweiligen Abmessungen des Lichtfeldes und des Strahlungsfeldes anzeigen, die bei dieser Einstellung ausgebildet werden; und Speichern der Daten als die Abmessungsdaten.

10. Verfahren nach Anspruch 9, bei welchem die Bestimmung der Abmessungsdaten das Kalibrieren des Strahlungsfeldes bei den mehreren Einstellungen der Feldabgrenzungsanordnung und das Vergleichen des Lichtfeldes mit Ergebnissen der Kalibrierung des Strahlungsfeldes umfasst.

11. Verfahren nach Anspruch 10, bei welchem die Kalibrierung des Strahlungsfeldes umfasst, bei jeder der Einstellungen einen angezeigten Wert für die Feldgröße des Strahlungsfeldes so anzupassen, dass er einem gemessenen Wert der Feldgröße des Strahlungsfeldes entspricht.

12. Verfahren nach Anspruch 9, 10 oder 11, bei welchem die Abmessungsdaten für jede der Einstellungen eine Differenz zwischen einer Abmessung des Lichtfeldes und der entsprechenden Abmessung des Strahlungsfeldes umfassen.

13. Verfahren nach Anspruch 9 oder 10 und 12, bei welchem der Vergleich umfasst, bei jeder der Einstellungen die Differenz zwischen dem angezeigten Wert für die Feldgröße und einem gemessenen Wert der Feldgröße des Lichtfeldes zu bestimmen, wodurch eine Grundlage zur Bestimmung der Differenz zwischen dem Lichtfeld und dem entsprechenden Strahlungsfeld bei jeder der Einstellungen der Feldabgrenzungsanordnung zur Verfügung gestellt wird.

14. Verfahren nach Anspruch 12 oder 13, bei welchem die Bestimmung der Differenz umfasst, die Abmessungsdaten so auszubilden und zu speichern, dass die Einstellungen der Feldabgrenzungsanordnung jeweiligen Differenzwerten zugeordnet sind, welche die Differenz zwischen den gemessenen Feldgrößen der entsprechenden Licht- und Strahlungsfelder angeben.

15. Verfahren nach Anspruch 14, bei welchem die Abmessungsdaten in Tabellenform gespeichert werden.

16. Verfahren nach einem der Ansprüche 9 bis 15, einsetzbar bei einer Strahleneinrichtung, bei welcher der Betriebsmodus mit einer von mehreren Strahlungsenergien betriebsbereit ist, so dass für jede Abmessung des Lichtfeldes, das in dem Einstellmodus ausgebildet wird, in dem Betriebsmodus mehrere entsprechende Abmessungen des Strahlungsfeldes für die unterschiedlichen Strahlenenergien vorhanden sind, wobei
die Bestimmung der Abmessungsdaten für mehrere Einstellungen der Feldabgrenzungsanordnung umfasst, Daten zu bestimmen, welche die jeweiligen Abmessungen des Lichtfeldes und mehrerer, entsprechender Strahlungsfelder anzeigen, die bei jeweiligen Strahlungsenergien ausgebildet werden.

17. Verfahren nach Anspruch 2 bis 5, 7 und 8, und Anspruch 6 in Abhängigkeit von Anspruch 2, bei welchem die Abmessungsdaten entsprechend dem Verfahren einer der Ansprüche 9 bis 16 bestimmt werden.

18. Verfahren nach einem der voranstehenden Ansprüche, bei welchem das Strahlungsfeld ein Röntgenstrahlungsfeld ist.

19. Strahlenbehandlungseinrichtung, bei welcher vorgesehen sind:
eine Strahlungsquelle (26) zur Erzeugung eines Strahlungsstrahls;
eine Vorrichtung (30, 34) zur Betätigung des Strahlungsstrahls zur Festlegung eines Strahlungsfelds in einer Behandlungszone (22);
eine Lichtquelle (52) zur Erzeugung eines Lichtstrahls;
eine Vorrichtung zur Betätigung des Lichtstrahls zur Festlegung eines Lichtfeldes in der Behandlungszone (22);
eine Feldabgrenzungsanordnung (36), die gemeinsam von der Strahlungs- und der Lichtstrahl-Betätigungsvorrichtung genutzt wird, und in dem Weg des Lichtstrahls und des Strahlungsstrahls angeordnet ist, wobei eine Einstellung der Feldabgrenzungsanordnung (36) eine Abmessung des Lichtfeldes und eine entsprechende Abmessung des Strahlungsfeldes festlegt; und
eine Vorrichtung zur Umschaltung der Strahleneinrichtung zwischen einem Einstellmodus, in welchem ein Lichtfeld in der Behandlungszone ausgebildet wird, und einem Betriebsmodus, in welchem ein Strahlungsfeld in der Behandlungszone ausgebildet wird;
**gekennzeichnet durch**
eine Vorrichtung (28, 60; 92) zur automatischen Anpassung der Einstellung der Feldabgrenzungsanordnung (36), wenn die Umschaltung zwischen dem Einstellmodus und dem Betriebsmodus erfolgt, wobei die Anpassung so ausgebildet ist, dass sie die Differenz der Abmessungen zwischen dem Lichtfeld und dem Strahlungsfeld kompensiert, die anderenfalls bei der Modusumschaltung aufträte, wenn die Einstellung der Feldabgrenzungsanordnung unverändert bliebe.

20. Einrichtung nach Anspruch 19, bei welcher die automatische Anpassungsvorrichtung (28) eine Speichervorrichtung (60) aufweist, in welcher Abmessungsdaten gespeichert sind, welche die Abmessungen und deren entsprechende Abmessungen in Abhängigkeit von der Einstellung der Feldabgrenzungsanordnung anzeigen.

21. Einrichtung nach Anspruch 20, bei welcher die Abmessungsdaten mehrere Differenzwerte umfassen, welche die jeweiligen Differenzen zwischen den Abmessungen und ihren entsprechenden Abmessungen anzeigen.

22. Einrichtung nach Anspruch 21, bei welcher die automatische Anpassungsvorrichtung so betreibbar ist, dass sie die Einstellung der Feldabgrenzungsanordnung um ein Ausmaß anpasst, das durch einen der Differenzwerte festgelegt ist.

23. Einrichtung nach Anspruch 21, bei welchem die automatische Anpassungsvorrichtung so betreibbar ist, dass sie die Einstellung der Feldabgrenzungsanordnung um ein Ausmaß anpasst, das durch Interpolation zwischen zumindest zweien der Differenzwerte festgelegt ist.

24. Einrichtung nach einem der Ansprüche 19 bis 23, die so betreibbar ist, dass sie ein Strahlungsfeld festlegt, das eine von mehreren Strahlungsenergien aufweist, wobei die automatische Anpassungsvorrichtung so betreibbar ist, dass sie die Einstellung der Feldabgrenzungsanordnung unter Berücksichtigung der Strahlungsenergie durchführt.

25. Einrichtung nach Anspruch 24 in Abhängigkeit von Anspruch 20, bei welcher die Abmessungsdaten mehrere Datenuntergruppen für jeweilige Strahlungsenergien aufweisen.

26. Einrichtung nach einem der Ansprüche 19 bis 25, bei welcher die Feldabgrenzungsanordnung eine Vorrichtung (40, 42) zur Festlegung der Abmessung und der entsprechenden Abmessung aufweist, und eine Vorrichtung (44, 46) zur Festlegung einer weiteren Abmessung des Lichtfeldes und einer entsprechenden weiteren Abmessung des Strahlungsfeldes.

27. Einrichtung nach Anspruch 26, bei welcher die Abmessungsfestlegungsvorrichtung zwei Paare von Spannbacken (40, 42 und 44, 46) aufweist, ein Paar für jede Abmessungsfestlegungsvorrichtung, wobei die Paare von Spannbacken so angeordnet sind, dass sie sich quer zueinander erstrecken, und ein jeweilige Vorrichtung (56, 58) aufweisen, um den Abstand zwischen den Spannbacken des betreffenden Paares anzupassen.

28. Einrichtung nach einem der Ansprüche 19 bis 27, welche eine Anzeige (66) zum Anzeigen eines Bildes des Strahlungsfeldes und einer Angabe der erwarteten Größe des Strahlungsfeldes bei der momentanen Einstellung der Feldabgrenzungsanordnung aufweist.

29. Einrichtung nach einem der Ansprüche 19 bis 28, bei welcher der Strahlungsstrahl ein Röntgenstrahlungsstrahl ist, und die Strahlungsstrahl-Betätigungsvorrichtung einen Röntgenstrahl-Kollimator aufweist, wobei die Licht- und die Strahlungsstrahl-Betätigungsvorrichtung so angeordnet sind, dass der jeweilige Weg des Lichtstrahls und des Röntgenstrahls so ausrichtet ist, dass das Licht entlang einer Achse gerichtet wird, die im wesentlichen koaxial zu jener des Röntgenstrahlungsstrahls verläuft, und durch die Feldabgrenzungsanordnung (36).

30. Einrichtung nach einem der Ansprüche 19 bis 29, bei welcher die automatische Anpassungsvorrichtung einen gekrümmten Spiegel (92) aufweist.

31. Einrichtung nach Anspruch 30, bei welchem der gekrümmte Spiegel (92) ein parabelförmiges Profil aufweist.

## Revendications

1. Méthode pour faire fonctionner un dispositif de radiothérapie ayant une structure définissant un champ, dans laquelle un réglage de la structure définissant un champ définit une dimension d'un champ lumineux formé dans un mode configuration du système et une dimension correspondante d'un champ de rayonnement formé dans un mode opérationnel du dispositif, la méthode comprenant l'ajustement automatique du réglage de la structure définissant un champ lorsqu'elle passe du mode configuration au mode opérationnel, l'ajustement étant configuré pour compenser la différence de dimension entre le champ lumineux et le champ de rayonnement qui autrement pourrait se produire lors du changement de mode si le réglage de la structure définissant un champ restait inchangé.

2. Méthode selon la revendication 1, dans laquelle ledit ajustement automatique se fait sur la base de données dimensionnelles stockées par le système, lesdites données dimensionnelles donnant une indication desdites dimensions et de leurs dimensions correspondantes en fonction du réglage de la structure définissant un champ.

3. Méthode selon la revendication 2, dans laquelle les données dimensionnelles comprennent une pluralité de valeurs de différence donnant une indication des différences respectives entre les dimensions et leurs dimensions correspondantes.

4. Méthode selon la revendication 3, dans laquelle l'ajustement automatique se fait en ajustant le réglage de la structure définissant un champ d'une quantité définie par l'une desdites valeurs de différence.

5. Méthode selon la revendication 3, dans laquelle l'ajustement automatique se fait en ajustant le réglage de la structure définissant un champ d'une quantité définie par interpolation entre au moins deux desdites valeurs de différence.

6. Méthode selon l'une quelconque des revendications précédentes, dans laquelle le système est tel que, en mode opérationnel, une parmi une pluralité d'énergies de rayonnement peut être opérante, la méthode étant telle que l'ajustement automatique du réglage de la structure définissant un champ tient compte de l'énergie de rayonnement.

7. Méthode selon la revendication 6 associée à la revendication 2, dans laquelle les données dimensionnelles comprennent une pluralité de sous-ensembles de données pour des énergies de rayonnement respectives.

8. Méthode selon l'une quelconque des revendications précédentes, dans laquelle l'ajustement automatique de la structure définissant un champ comprend l'ajustement automatique des mâchoires d'un collimateur qui est relié à un accélérateur linéaire.

9. Méthode pour déterminer des données dimensionnelles pour un dispositif de radiothérapie ayant une structure définissant un champ, dans laquelle un réglage de la structure définissant un champ définit une dimension d'un champ lumineux formé dans un mode configuration du dispositif et une dimension correspondante d'un champ de rayonnement formé dans un mode opérationnel du dispositif, la méthode comprenant les étapes consistant à :
déterminer, pour une pluralité de réglages de la structure définissant un champ, des données donnant une indication de la différence entre des dimensions respectives du champ lumineux et le champ de rayonnement formé à ce réglage ; et
stocker lesdites données en tant que lesdites données dimensionnelles.

10. Méthode selon la revendication 9, dans laquelle la détermination des données dimensionnelles comprend l'étalonnage du champ de rayonnement à la pluralité de réglages de la structure définissant un champ et la comparaison du champ lumineux avec les résultats d'étalonnage du champ de rayonnement.

11. Méthode selon la revendication 10, dans laquelle l'étalonnage du champ de rayonnement comprend, à chacun des réglages, l'ajustement d'une valeur affichée de la taille de champ du champ de rayonnement pour qu'elle corresponde à une valeur mesurée de la taille de champ du champ de rayonnement.

12. Méthode selon l'une des revendications 9, 10 ou 11, dans laquelle lesdites données dimensionnelles comprennent, pour chacun desdits réglages, une différence entre une dimension du champ lumineux et la dimension correspondante du champ de rayonnement.

13. Méthode selon la revendication 10 ou la revendication 11 et la revendication 12, dans laquelle la comparaison comprend, à chacun desdits réglages, la détermination de la différence entre la valeur affichée de la taille de champ et une valeur mesurée de la taille de champ du champ lumineux, fournissant ainsi une base pour déterminer ladite différence entre le champ lumineux et le champ de rayonnement correspondant à chacun desdits réglages de la structure définissant un champ.

14. Méthode selon la revendication 12 ou la revendication 13, dans laquelle la détermination de ladite différence comprend la formation et le stockage des données dimensionnelles de telle façon que les réglages de la structure définissant un champ sont associés à des valeurs de différence respectives donnant une indication de la différence entre les tailles de champ mesurées des champs lumineux et de rayonnement correspondants.

15. Méthode selon la revendication 14, dans laquelle les données dimensionnelles sont stockées sous forme tabulaire.

16. Méthode selon l'une quelconque des revendications 9 à 15, applicable à un dispositif de rayonnement dans lequel le mode opérationnel est opérant avec une parmi une pluralité d'énergies de rayonnement de telle façon que, pour une dimension quelconque du champ lumineux formé en mode configuration, il y a en mode opérationnel une pluralité de dimensions correspondantes du champ de rayonnement pour les différentes énergies de rayonnement, dans laquelle la détermination des données dimensionnelles pour une pluralité de réglages de la structure définissant un champ comprend la détermination de données donnant une indication des dimensions respectives du champ lumineux et une pluralité de champs de rayonnement correspondants formés à des énergies de rayonnement respectives.

17. Méthode selon les revendications 2 à 5, les revendications 7 et 8 et la revendication 6 associée à la revendication 2, dans laquelle les données dimensionnelles sont déterminées selon la méthode de l'une quelconque des revendications 9 à 16.

18. Méthode selon l'une quelconque des revendications précédentes, dans laquelle ledit champ de rayonnement est un champ de rayonnement des rayons X.

19. Dispositif de radiothérapie comprenant :
une source de rayonnement (26) pour générer un faisceau de rayonnement ;
des moyens (30, 34) pour manipuler le faisceau de rayonnement afin de définir un champ de rayonnement dans une zone de traitement (22) ;
une source lumineuse (52) pour générer un faisceau lumineux ;
des moyens pour manipuler le faisceau lumineux afin de définir un champ lumineux dans la zone de traitement (22) ;
une structure définissant un champ (36) commune aux moyens de manipulation d'un champ de rayonnement et d'un champ lumineux et agencée dans le trajet du faisceau lumineux et du faisceau de rayonnement, un réglage de la structure définissant un champ (36) qui définit une dimension du champ lumineux et une dimension correspondante du champ de rayonnement ; et
des moyens pour faire passer le dispositif de rayonnement du mode configuration, dans lequel un champ lumineux est formé dans la zone de traitement, en mode opérationnel, dans lequel un champ de rayonnement est formé dans la zone de traitement ; **caractérisé par**
des moyens (28, 60, 92) pour ajuster automatiquement le réglage de la structure définissant un champ (36) lorsqu'elle passe du mode configuration au mode opérationnel, l'ajustement étant configuré pour compenser la différence de dimension entre le champ lumineux et le champ de rayonnement qui autrement pourrait se produire lors du changement de mode si le réglage de la structure définissant un champ restait inchangé.

20. Dispositif selon la revendication 19, dans lequel les moyens d'ajustement automatique (28) possèdent des moyens de stockage (60) dans lesquels sont stockées des données dimensionnelles donnant une indication desdites dimensions et de leurs dimensions correspondantes en fonction du réglage de la structure définissant un champ.

21. Dispositif selon la revendication 20, dans lequel les données dimensionnelles comprennent une pluralité de valeurs de différence donnant une indication des différences respectives entre les dimensions et leurs dimensions correspondantes.

22. Dispositif selon la revendication 21, dans lequel les moyens d'ajustement automatique sont opérables pour ajuster le réglage de la structure définissant un champ d'une quantité définie par l'une desdites valeurs de différence.

23. Dispositif selon la revendication 21, dans lequel les moyens d'ajustement automatique sont opérables pour ajuster le réglage de la structure définissant un champ d'une quantité définie par interpolation entre au moins deux desdites valeurs de différence.

24. Dispositif selon l'une quelconque des revendications 19 à 23, opérable pour définir un champ de rayonnement ayant une parmi une pluralité d'énergies de rayonnement, les moyens d'ajustement automatique étant opérables pour effectuer le réglage de la structure définissant un champ en tenant compte de l'énergie de rayonnement.

25. Dispositif selon la revendication 24 associée à la revendication 20, dans lequel les données dimensionnelles comprennent une pluralité de sous-ensembles de données pour des énergies de rayonnement respectives.

26. Dispositif selon l'une quelconque des revendications 19 à 25, dans lequel la structure définissant un champ comprend des moyens (40, 42) pour définir ladite dimension et ladite dimension correspondante et des moyens (44, 46) pour définir une autre dimension du champ lumineux et une autre dimension correspondante du champ de rayonnement.

27. Dispositif selon la revendication 26, dans lequel lesdits moyens définissant une dimension comprennent deux paires de mâchoires (40, 42 et 44, 46), une paire pour chaque moyen définissant une dimension, les paires de mâchoires étant agencées de façon à s'étendre transversalement l'une par rapport à l'autre et ayant des moyens respectifs (56, 58) pour ajuster la séparation entre les mâchoires de la paire concernée.

28. Dispositif selon l'une quelconque des revendications 19 à 27 et comprenant un afficheur (66) pour afficher une image du champ de rayonnement et une indication de la taille attendue du champ de rayonnement pour le réglage courant de la structure définissant un champ.

29. Dispositif selon l'une quelconque des revendications 19 à 28, dans lequel le faisceau de rayonnement est un faisceau de rayonnement de rayons X et les moyens de manipulation du faisceau de rayonnement comprennent un collimateur de rayons X, les moyens de manipulation du faisceau lumineux et du faisceau de rayonnement étant agencés de façon que les trajets respectifs du faisceau lumineux et du faisceau de rayons X sont alignés de façon à diriger le faisceau lumineux le long d'un axe qui est généralement coaxial à celui du faisceau de rayonnement de rayons X et traverse ladite structure définissant un champ (36).

30. Dispositif selon l'une quelconque des revendications 19 à 29, dans lequel les moyens d'ajustement automatique comprennent un miroir courbe (92).

31. Dispositif selon la revendication 30, dans lequel le miroir courbe (92) a un profil parabolique.
